(19) Europäisches Patentamt
European Patent Office
Office européen des brevets

(11) Veröffentlichungsnummer: **0 008 341**
**B1**

# (12) EUROPÄISCHE PATENTSCHRIFT

(45) Veröffentlichungstag der Patentschrift: **04.11.81**

(51) Int. Cl.³: **C 12 Q 1/00**

(21) Anmeldenummer: **79102248.6**

(22) Anmeldetag: **03.07.79**

(54) **Verfahren und Reagenz zur Durchführung enzymatischer Bestimmungen und Testkit zur Durchführung des Verfahrens.**

(30) Priorität: **08.08.78 DE 2834705**

(43) Veröffentlichungstag der Anmeldung: **05.03.80 Patentblatt 80/5**

(45) Bekanntmachung des Hinweises auf die Patenterteilung: **04.11.81 Patentblatt 81/44**

(84) Benannte Vertragsstaaten: **BE CH DE FR GB IT LU NL SE**

(56) Entgegenhaltungen:
**DE-A-1 959 410**
**DE-A1-2 518 862**
**DE-A1-2 601 961**
**US-A-3 985 621**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Danninger, Josef, Finkenstrasse 27a, D-8032 Gräfelfing (DE)**
Erfinder: **Deneke, Ulfert, Dr., Ammerhöfe 80 1/2, D-8123 Peissenberg (DE)**

(74) Vertreter: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

EP 0 008 341 B1

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren und Reagenz zur Durchführung enzymatischer Bestimmungen und Testkit zur Durchführung des Verfahrens

Die Erfindung betrifft ein Verfahren zur Bestimmung von Substraten oder Enzymen in Serum, Körperflüssigkeiten oder anderem Probenmaterial, bei denen die Bildung von NADH oder NADPH Meßsignal ist.

In der klinischen und pharmazeutischen Chemie, der Biochemie und der Lebensmittelchemie sind enzymatische Reaktionen, die unter NADH- oder NADPH-Bildung verlaufen, von großem Interesse, weil sie in der Regel durch gute Spezifität und rasche Durchführbarkeit auszeichnen. Liegt eine ausreichend spezifische enzymatische Reaktionskette vor, so sind Störungen nur durch solche Substanzen zu erwarten, die als Zwischenprodukte der Hauptreaktion auftreten können. Unspezifitäten der Hilfs- und Indikatorenzyme können ebenfalls Störstoffe aus der Probe zur Reaktion bringen. Es ist daher üblich, Leerwerte mitzuführen, um den Einfluß dieser Substanzen bei der Messung zu berücksichtigen. Man muß jedoch hinnehmen, daß dadurch ein Teil des Meßbereichs bereits verbraucht wird. Ohne Verstellen eines Photometers sind normalerweise nur Extinktionen bis zu 2 zu messen. Liegt in einer Probe nun die Störstoffkonzentration sehr hoch, so kann bei jeder Messung ein Umschalten oder Umstellen des Photometers erforderlich sein. Noch schwieriger ist die Lage, wenn das Verhältnis von Störstoffkonzentration zu eigentlicher Meßkonzentration groß ist. Dann ist der eigentliche Meßwert die kleine Differenz zweier großer Meßsignale. Solche Meßverfahren sind mit untragbar großen Fehlern belastet, da sich auch kleinste Meßfehler in den großen Meßsignalen stark auswirken und unerfüllbare Anforderungen an die Pipettiergenauigkeit der Probe und die Meßgenauigkeit des Geräts stellen.

Bei Bestimmungen von Enzymaktivitäten wirkt sich die Anwesenheit von Störstoffen der Hilfs- und Indikatorreaktionen in der Probe in der Weise aus, daß vor der eigentlichen, linear verlaufenden Reaktion ein Vorlauf gemessen wird. Auch hier kann der Meßbereich durch den Vorlauf bereits stark eingeschränkt sein, wie oben beschrieben. Hier kann aber auch eine weitere Störung zum Tragen kommen. Viele Enzymaktivitäten zeigen eine sogenannte Lagphase, d. h. es dauert eine gewisse Zeit, bis die maximale Reaktionsgeschwindigkeit erreicht wird. Die Messung kann dann erst nach Ablauf dieser Lag-phase vorgenommen werden. Auch hierbei wird ein Teil des ohne Veränderung des Photometers zur Verfügung stehenden Meßbereichs verbraucht. Dazu kommt, daß viele Analysenautomaten nicht in der Lage sind, nach Start durch Zugabe der Probe mit der Messung zu warten, bis die Lag-phase abgelaufen ist. Auf solche Automaten können enzymatische Meßverfahren, die einen Vorlauf, eine Lag-phase oder beides zeigen, nicht adaptiert werden.

Prinzipiell könnte die Zerstörung von Störstoffen mit einer anderen Reaktion als der Meßreaktion Abhilfe schaffen.

In der DE-AS 2 518 862 wird ein Verfahren beschrieben, bei dem Lactatdehydrogenase, welche die Messung NADH bildender Reaktionen im Serum stört, durch Oxalat oder Oxamat gehemmt wird. Im Verfahren der US-PS 3 985 621 werden mit Hilfe eines spezifischen Inhibitors Dehydrogenasereaktionen abgestoppt, ohne daß die Messung der NAD(P)H-Absorption bei 340 mμ beeinflußt wird.

Störende Glucose kann bei der Saccharose-Bestimmung durch Glucose-oxidase beseitigt werden (H. U. Bergmeyer, Methoden der enzymatischen Analyse, 2. Auflage, Verlag Chemie, Weinheim, 1970, Bd. II, Seite 1145). Im Amylase-Test kann die endogene Glucose und die während der Lag-phase gebildete Glucose ebenfalls durch Glucose-oxidase beseitigt werden (Clin. Chem. 23, 1149 [1977]). In jedem Fall wird anschließend die bei der eigentlich interessierenden Meßreaktion freigesetzte Glucose über NADH- oder NADPH-Bildung im Hexokinase-/-Glucose-6-phosphat-dehydrogenasesystem gemessen. Es ist jedoch offensichtlich, daß diese Methode nicht auf Reaktionen angewendet werden kann, bei denen andere Stoffe als Glucose stören. Wie schon aufgeführt wurde, werden jedoch prinzipiell alle enzymatischen Meßketten durch das Auftreten von Zwischenprodukten in der Probe gestört.

Ziel dieser Erfindung ist es daher, ein Verfahren zu schaffen, mit dessen Hilfe NADH- und NADPHliefernde Reaktionen so geführt werden können, daß weder das Auftreten von Zwischenprodukten noch von Substraten für Fremd- und Nebenaktivitäten der Hilfs- und Indikatorenzyme in der Probe stören. Gleichzeitig soll dieses Verfahren geeignet sein, bei Enzymmessungen etwa auftretende Lag-phasen zu eliminieren.

Gelöst wird diese Aufgabe erfindungsgemäß durch ein Verfahren zur Durchführung enzymatischer Bestimmungen, die unter Bildung von NAD(P)H verlaufen und bei denen Zwischenprodukte und unspezifisch reagierende Störsubstanzen in einer Vorreaktion abreagieren gelassen werden, welches dadurch gekennzeichnet ist, daß man während der Vorreaktion gleichzeitig eine NAD(P)H-verbrauchende enzymatische Reaktion mitführt, die das gebildete NAD(P)H vollständig zu NAD(P) oxidiert und zum Start der Meßreaktion die NAD(P)H-verbrauchende Reaktion gestoppt wird.

Erfindungsgemäß wird also während der Vorreaktion, in der Zwischenprodukte und unspezifisch reagierende Störsubstanzen abreagieren und gegebenenfalls bei Enzymreaktionen die Lag-phase abläuft, eine NADH- oder NADPH-verbrauchende Reaktion mitgeführt, die alles gebildete NADH oder NADPH oxidiert.

Es ist überraschend, daß es möglich ist, die Erfindung in der angegebenen Weise zu verwirklichen. Es war nicht vorhersehbar, daß die gleichzeitige Durchführung zweier gegenläufiger Reaktionen ohne Schwierigkeiten möglich ist. Außerdem war damit zu rechnen, daß das benötigte zweite Substrat, der Inhibitor und die Substanz, die zur Zerstörung des zweiten Substrats angewandt wird, die eigentliche Meßreaktion beeinflussen würde, da enzymatische Reaktionen außerordentlich empfindlich auf Änderungen der Reaktionsbedingungen reagieren. Sodann war nicht zu erwarten, daß die Gegenreaktion überhaupt unter den Bedingungen der Meßreaktion ablaufen würde, die ja in der Regel weit von den optimalen Bedingungen der Gegenreaktion abweichen und nicht verändert werden dürfen.

Die Reaktionsbedingungen bezüglich pH-Wert, Puffer, Reagenskonzentrationen usw. sind für diese Reaktionen ebenso wie für die Hauptreaktion dem Fachmann bekannt und unter Berücksichtigung dieser Bedingungen lassen sich die jeweils bestgeeigneten NAD(P)H-verbrauchenden Reaktionen auswählen.

Der Start der eigentlichen Meßreaktion erfolgt, indem die NAD(P)H-verbrauchende Reaktion gestoppt wird, was durch Zufügen eines Inhibitors, der die NADH- bzw. NADPH-verbrauchende Reaktion vollständig hemmt, oder einer Substanz, die das zweite Substrat der NADH- oder NADPH-verbrauchenden Reaktion momentan vollständig umsetzt, erfolgen kann. Handelt es sich um eine Substratmessung, so wird gleichzeitig das Startenzym zugesetzt. Enzymreaktionen dagegen erfordern diese Zugabe nicht, sondern können sofort abgelesen werden. Der Startzeitpunkt kann frei gewählt werden und wird so ausgesucht, daß sämtliche Störreaktionen bereits vollständig abgelaufen sind.

Prinzipiell können im Rahmen des erfindungsgemäßen Verfahrens alle NADH- und NADPH-verbrauchenden Reaktionen eingesetzt werden, wenn entweder ein geeigneter Inhibitor zur Verfügung steht oder eine Reaktion, die das zweite Substrat praktisch momentan umsetzt. Eine Anzahl von Beispielen für derartige, im Rahmen der Erfindung geeignete Gegenreaktionen sind in der nachstehenden Tabelle aufgeführt.

Tabelle 1

| Enzym (EC-Nr.) | Substrat der Gegenreaktion | Abstoppen der Gegenreaktion | |
|---|---|---|---|
| | | Inhibitor | Abreaktion des 2. Substrats |
| Alkohol-dehydrogenase 1.1.1.1 | NADH/verschiedene Aldehyde | NADP, AMP, ADP | Ketonfänger wie Hydrazin, Hydrazone, Semicarbazide |
| dto. 1.1.1.2 | NADPH/verschiedene Aldehyde | | Ketonfänger |
| L-Xylulose-reduktase 1.1.1.10 | NADPH/Xylulose | Xyluloseüberschuß | |
| L-Iditol-dehydrogenase = Sorbitdehydrogenase 1.1.1.14 | NADH/Fructose | 2,3-Dithio-1-propanol | |
| Mannitol-1-phosphat-dehydrogenase 1.1.1.17 | NADH/Fructose-6-phosphat | | Phosphatase |
| Aldose-reduktase 1.1.1.21 | NAD(P)H/verschiedene Aldosen und Aldehyde | α-KG, Acetacetat, L-Oxovalerat | |
| Glyoxylat-reduktase 1.1.1.26 | NADH/Glyoxylat (Hydroxypyruvat) | | Ketonfänger |
| Lactatdehydrogenase 1.1.1.27 | NADH/Pyruvat | LDH-Inhibitor Fluoropyruvat, Oxalat | Ketonfänger |
| Glycerat-dehydrogenase 1.1.1.29 | NAD(P)H/Hydroxypyruvat | | Ketonfänger |
| 3-Hydroxybutyrat-DH 1.1.1.30 | NADH/Acetoacetat | D-Lactat | |
| Oxaloglycerat-reductase 1.1.1.92 | NAD(P)H/Dihydroxyfumarat | | $SO_3^{2-}$ |
| Dijodo-phenylpyruvat-reductase 1.1.1.96 | NADH/Dijodophenylpyruvat | 2,5-Dihydroxybenzoat | |
| Mannitol-dehydrogenase 1.1.1.138 | NADP/Fructose | 2,3-Dithiol-1-propanol | |
| Glyoxalat-dehydrogenase 1.2.1.17 | NADPH/Oxaloyl-CoA | Tris | |
| Alanin-dehydrogenase 1.4.1.1 | NADH/Pyruvat/$NH_3$ | Ketonsäuren | Ketonfänger |
| GIDH 1.4.1.3 | NADH(P)H/α-/-$NH_3$ | GPT | Ketonfänger |
| D-Prolin-reductase 1.4.1.6 | NADH/D-Prolin | 5-Aminovalerat | |
| Pyrolin-2-carbonsäure-reductase 1.5.1.1 | NADH/Pyrolin-2-carbonsäure | 2-Aminooctanoat | |
| Glutathion-reduktase 1.6.4.2 | NAD(P)H/GSSG | Thiocid, 5-Nitrofuraldehyd-p-Nitrobenzolsulfonsäure | Dithiothreitol |
| Hydroxylamin-reduktase 1.7.99.1 | NAD(P)H/Hydroxylamin | Hydrazin | Keton oder Aldehyd |
| NADH-POD 1.11.1.1 | NADH/$H_2O_2$ | Bromid | Katalase Ascorbat $SO_3^{2-}$ Brenzcatechin/Peroxidase Glutathion/Glutathionperoxidase |

Besonders bevorzugt werden folgende NADH- bzw. NADPH-verbrauchende Reaktionen:

1. $NADH + Pyruvat + H^{+} \xrightarrow{LDH} Lactat + NAD^{+}$
   LDH = Lactatdehydrogenase

Der Start erfolgt mit LDH-Inhibitor.

2. Fructose + NADH + $H^+$ $\xrightarrow{SDH}$ Sorbit + $NAD^+$
SDH = Sorbitdehydrogenase

Der Start erfolgt mit 2,3-Dithiol-1-propanol als SDH-Inhibitor.

3. Diglutathiondisulfid + NAD(P)H + $H^+$ $\xrightarrow{GR}$ 2 Glutathion + $NAD(P)^+$
GR = Glutathionreduktase

Der Start erfolgt mit Dithiothreitol, das momentan das GSSG zu GSH reduziert.

4. $H_2O_2$ + NADH + $H^+$ $\xrightarrow{NADH-POD}$ 2 $H_2O$ + $NAD^+$
NADH–POD = NADH-Peroxidase.

Der Start kann entweder mit Katalase oder mit $HSO_3^-$ oder mit Glutathion/Glutathion-peroxidase erfolgen, die das zweite Substrat $H_2O_2$ momentan zerstören.

Grundsätzlich eignet sich das erfindungsgemäße Verfahren für alle unter NAD(P)H-Bildung verlaufenden Enzym- und Substratbestimmungen. Typische Beispiele sind in der nachstehenden Tabelle 2 unter Angabe der Enzym/Substrat-Paare angeführt. Jedoch können auch Enzymaktivitäten oder Substrate in gekoppelter Reaktion eingesetzt werden, wenn sie unter NAD(P)H-Bildung verlaufen. Dies wird unten in den Beispielen noch näher erläutert.

Tabelle 2

| Enzym | Substrat | In gekoppelter Reaktion nachweisbar | |
|---|---|---|---|
| | | Enzym | Substrat |
| 1.1.1.21 UDP-Glucose-dehydrogenase | UDP-Glucose/NAD | UDP-Glucose-4-epimerase, Saccharose-synthetase | ATP, ADP, UTP, UDP, CMP, UMP, UDP-Glucose, Saccharose |
| 1.1.1.41 Isocitrat-dehydrogenase | Threo-isocitrat NAD | | |
| 1.1.1.44 Phosphoglyconat-dehydrogenase | Phosphogluconat/ NAD(P) | Creatinkinase, Pyruvatkinase, Hexokinase, α-Amylase, α-Glucosidase, β-Gluosidase | Glucose, Fructose, Maltose, ATP, Creatinphosphat, Oligosaccharide, Stärke |
| 1.1.1.48 Galactose-dehydrogenase | Galactose/NAD | α-Glactosidase, β-Glactosidase | Lactose, Galactose-1-phosphat Galactose-6-phosphat |
| 1.1.1.49 Glucose-6-phosphat-dehydrogenase | Glucose-6-phosphat/ NAD(P) | Creatinkinase, Pyruvatkinase, Hexokinase, α-Amylase, α-Glucosidase, β-Glucosidase | Glucose, Fructose, Maltose, Oligosaccharide, Stärke |
| 1.2.1.1 Formaldehyd-dehydrogenase | Formaldehyd/NAD | | Methanol |
| 1.2.1.2 Formiat-dehydrogenase | Formiat/NAD | | Oxalat, Methanol, Formaldehyd, Sarcosin |
| 1.2.1.3—1.2.1.5 Aldehyd-dehydrogenase | Acetaldehyd/NAP(P) | Alkohol-oxidasen, Alkohol-DH, Diamino-oxidase | verschiedene Alkohole und Aldehyde, Glycerin |
| 1.2.1.14 IMP-Dehydrogenase | Inosin-5-phosphat/ NAD | Creatinkinase, Myokinase | AMP, ADP, GMP, CMP, UMP |
| 1.2.1.16 und 1.2.1.24 Succinat-semialdehyd-dehydrogenase | Succinat-semi-aldehyd/NAD(P) | GOT, GPT, Pyruvat-kinase, GIDH | ADP, Creatinin, α-KG, Pyruvat, Oxalacetat, γ-Aminobutyrat, Alanin, Glutamat |

Ein weiterer Gegenstand der Erfindung ist ein Reagens zur Durchführung enzymatischer Bestimmungen, enthaltend ein System zur Bestimmung eines Substrats oder Enzyms, welches zur Bildung von NAD(P)H führt, welches dadurch gekennzeichnet ist, daß es zusätzlich ein enzymatisches System enthält, welches NAD(P)H vollständig zu NAD(P) oxidiert.

Ein weiterer Gegenstand der Erfindung ist ein Testkit zur Durchführung des erfindungsgemäßen Verfahrens, welcher aus dem oben beschriebenen, erfindungsgemäßen Reagens und einem Abstoppmittel für die NAD(P)H-verbrauchende Reaktion besteht.

Wie bereits erwähnt, wird es durch die Erfindung möglich, Fremd- und Nebenaktivitäten bei den durchzuführenden Bestimmungsmethoden auszuschalten und Lag-phasen zu eliminieren. Dies hat zur Folge, daß derartige Reaktionen auch auf Analysenautomaten durchgeführt werden können, welche an sich nicht für enzymatische Meßverfahren geeignet sind, die einen Vorlauf oder eine Lag-phase zeigen. Außerdem werden auch noch die Meßsignale genauer und besser bestimmbar, insbesondere bei hohen Störstoffkonzentrationen.

In den nachfolgenden Beispielen werden die folgenden Abkürzungen verwendet:

| | |
|---|---|
| GSSG | Glutathion, oxidiert |
| SDH | Sorbit-dehydrogenase |
| GR | Glutathion-reductase |

| | |
|---|---|
| GSH | Glutathion |
| Glyc-DH | Glycerin-dehydrogenase |
| Al-DH | Aldehyd-dehydrogenase |
| Gluc-DH | Glucose-dehydrogenase |
| GPT | Glutamat-pyruvat-transaminase |
| $\alpha$-KG | $\alpha$-Ketoglutarat |
| GAB-GT | $\gamma$-Aminobutyrat-$\alpha$-ketoglutarat-transaminase |
| SS-Al-DH | Succinatsemialdehyd-dehydrogenase |
| ADP | Adenosin-5'-diphosphat |
| AMP | Adenosin-monophosphat |
| ATP | Adenosin-5'-triphosphat |
| PEP | Phosphoenol-pyruvat |
| PK | Pyruvat-kinase |
| NAD | Nicotinamid-adenin-denucleotid |
| NADH | Nicotinamid-adenin-dinucleotid, reduziert |
| NADP | Nicotinamid-adenin-dinucleotid-phosphat |
| NAD(P) | NADP oder NAD |
| NADPH | Nicotinamid-adenin-dinucleotidphosphat, reduziert |
| NAD(P)H | NADPH oder NADH |
| LDH | Lactat-dehydrogenase |
| CK | Creatin-kinase |
| NADH-POD | NADH-Peroxidase |
| HK | Hexokinase |
| G6P-DH | Glucose-6-phosphat-dehydrogenase |
| Tris | Tris(hydroxymethyl)-aminomethan |

2,3-Dithio-1-propanol = 2,3-Dithiol-1-propanol = 2,3-Dimercapto-1-propanol.

Die folgenden Beispiele erläutern die Erfindung weiter.

## Beispiel 1

Beseitigung von freiem Glycerin und störenden Aldehyden im Triglycerid-test.

A a) $\text{Triglycerid} + 3\ H_2O \xrightarrow{\text{Esterase}} 3\ \text{Fettsäuren} + \text{Glycerin}$

b) $\text{Glycerin} + NAD^+ \xrightarrow{\text{Glyc-DH}} \text{Glycerinaldehyd} + NADH + H^+$

c) $\text{Glycerinaldehyd} + NAD^+ \xrightarrow{\text{Al-DH}} \text{Glycerinsäure} + NADH + H^+$

können freies Glycerin und Aldehyde, die unspezifisch mit der Al-DH (=Aldehyddehydrogenase) reagieren, zu überhöhten Leerwerten führen. Diese Störung läßt sich wie folgt beseitigen:

$$NADH + \text{Pyruvat} + H^+ \xrightarrow{\text{LDH}} \text{Lactat} + NAD^+$$

Nachstehende Tabelle erläutert die Durchführung der Reaktion:

Tabelle 3

| Ausgangslösung | Probe | Leerwert | Konz. im Test |
|---|---|---|---|
| Tris · HCl, pH 8,3, 100 mMol/l | 1,5 ml | 1,5 ml | 50 mMol/l |
| NAD, 27 mMol/l | 0,1 ml | 0,1 ml | 0,9 mMol/l |
| Glycerin-DH, 100 U/ml | 0,1 ml | 0,1 ml | 3,3 U/ml |
| Al-DH, 200 U/ml | 0,05 ml | 0,05 ml | 3,3 U/ml |
| Pyruvat, 120 mMol/l | 0,05 ml | 0,05 ml | 2 mMol/l |
| Probe | 0,05 ml | — | |
| LDH, 100 U/ml | 0,05 ml | 0,05 ml | 1,65 U/ml |

In Probe und Leerwert reagieren nunmehr Glycerin und Aldehyde unter NADH-Bildung ab, welche

sofort durch Pyruvat und LDH wieder zu NAD oxidiert wird. Nach 5 min erfolgt der Start mit

| | | | |
|---|---|---|---|
| Esterase, 2000 U/ml | 0,1 ml | 0,1 ml | 33 U/ml |
| LDH-Inhibitor*), 20 mMol/l | | | 0,33 mMol/l |

*) B.B.A. 54, 210 (1961).

Der LDH-Inhibitor hemmt die LDH nunmehr vollständig. Die Esterase spaltet aus den Triglyceriden Glycerin ab, das unter NADH-Bildung weiter reagiert. Das NADH bleibt unverändert, da die LDH nicht mehr aktiv ist. Nach 5 min kann daher die Extinktionsdifferenz zwischen Probe und Leerwert gemessen und daraus der Triglyceridgehalt berechnet werden.

Die Reaktionsbedingungen dieses Tests werden durch die optimalen Bedingungen für den Triglycerid-test bestimmt. Erfindungsgemäß ist lediglich der Zusatz von Pyruvat, LDH und LDH-Inhibitor. Die Pyruvat-konzentration wird durch die Menge der zu beseitigenden Störsubstanzen bestimmt und kann zwischen 0,005 mMol/l und 100 mMol/l schwanken. Bevorzugt sind 0,5 bis 5 mMol/l. Die Konzentration der LDH wird durch die Inkubationszeit bestimmt. Sie kann zwischen 0,001 U/ml bis 100 U/l schwanken. Bevorzugt sind 0,5 bis 5 U/ml. Die Mindestkonzentration des LDH-Inhibitors muß ausreichen, um die LDH quantitativ zu hemmen. Das ist bei 0,2 mMol/l der Fall. Höhere Konzentrationen mit 5 mMol/l stören nicht. Bevorzugt sind 0,2 bis 2 mMol/l.

## Beispiel 2

Beseitigung von störendem Creatin, ADP, Pyruvat und α-KG in der Creatinin-bestimmung.
Bei der Bestimmung von Creatinin im Serum nach folgendem Schema

B a) Creatinin + $H_2O$ $\xrightarrow{\text{Creatininase}}$ · Creatin

b) Creatin + ATP $\xrightarrow{\text{CK}}$ Creatinphosphat + ADP

c) ADP + PEP $\xrightarrow{\text{PK}}$ Pyruvat + ATP

d) Pyruvat + Glutamat $\xrightarrow{\text{GPT}}$ α-KG + Alanin

e) α-KG + 4 Aminobutyrat $\xrightarrow{\text{GAB-GT}}$ Succinat-semialdehyd + Glutamat

f) Succinat-semialdehyd + $NAD^+$ $\xrightarrow{\text{SS-Al-DH}}$ Succinat + NADH + $H^+$

stören aus dem Serum stammendes Creatin, Pyruvat und α-KG sowie vor allem das im ATP vorhandene ADP, weil sie zu hohe Leerwerte ergeben. Die Störung wird wie folgt beseitigt:

g) NADH + Fructose + $H^+$ $\xrightarrow{\text{SDH}}$ Sorbit + $NAD^+$

Die Durchführung der Reaktion erläutert folgende Tabelle.

Tabelle 4

| Ausgangslösung | Probe | Leerwert | Konz. im Test |
|---|---|---|---|
| Glycin-puffer, 0,2 Mol/l, pH 8,0 | 1,0 ml | 1,0 ml | 0,1 Mol/l |
| NADP, 80 mMol/l | 0,05 ml | 0,05 ml | 2 mMol/l |
| ATP, 40 mMol/l | 0,05 ml | 0,05 ml | 1 mMol/l |
| PEP, 16 mMol/l | 0,05 ml | 0,05 ml | 0,4 mMol/l |
| Glutamat, 44 mMol/l | 0,05 ml | 0,05 ml | 1,1 mMol/l |
| $\gamma$-Aminobutyrat, 380 mMol/l | 0,5 ml | 0,5 ml | 95 mMol/l |
| Magnesiumchlorid, 40 mMol/l | 0,1 ml | 0,1 ml | 2 mMol/l |
| Fructose, 80 mMol/l | 0,05 ml | 0,05 ml | 2 mMol/l |
| CK, 400 U/ml | 0,05 ml | 0,05 ml | 10 U/ml |
| PK, 160 U/ml | 0,05 ml | 0,05 ml | 4 U/ml |
| SDH, 20 U/ml | 0,05 ml | 0,05 ml | 0,5 U/ml |
| GPT, 160 U/ml | 0,05 ml | 0,05 ml | 4 U/ml |
| GAB-GT, 160 U/ml | 0,05 ml | 0,05 ml | 4 U/ml |
| SS-Al-DH, 160 U/ml | 0,05 ml | 0,05 ml | 4 U/ml |
| Probe | 0,5 ml | — | |
| $H_2O$ | — | 0,5 ml | |

10 min inkubieren. Während dieser Zeit werden Creatin, Pyruvat, $\alpha$-KG und ADP umgesetzt. Das gebildete NADH wird durch Fructose/SDH beseitigt. Dann Start mit

| | | | |
|---|---|---|---|
| Creatinase, 400 U/ml | 0,05 ml | 0,05 ml | 10 U/ml |
| 2,3-Dithiol-1-propanol, 80 mMol/l | | | 2 mMol/l |

Durch den Start mit Creatinase kommt die Creatininbestimmung in Gang. Der Inhibitor 2,3-Dithiol-1-propanol hemmt die SDH vollständig, so daß nunmehr die aus der Creatiniumsetzung stammende NADH-Bildung gemessen werden kann.

Die Reaktionsbedingungen sind durch die optimierte Creatininbestimmung festgelegt. Für den erfindungsgemäßen Zusatz von Fructose, SDH und 2,3-Dithiol-1-propanol gilt das unter Beispiel 1 gesagte. Dementsprechend kann die Fructose-konzentration zwischen 0,002 bis 200 mMol/l liegen, bevorzugt sind 0,5 bis 10 mMol/l. SDH kann zwischen 0,002 bis 50 U/ml schwanken, bevorzugt sind 0,1 bis 5 U/ml. Für 2,3-Dithiol-1-propanol gelten 0,1 bis 100 mMol/l als Grenzen, bevorzugt sind 1 bis 10 mMol/l.

## Beispiel 3

Beseitigung einer Lag-phase in der Creatinkinase-bestimmung.
Bei der Bestimmung von CK im Serum, beruhend auf Schema

C a) Creatinin-phosphat + ADP $\xrightarrow{CK}$ ATP + Creatinin

b) ATP + Glucose $\xrightarrow{HK}$ Glucose-6-phosphat + ADP

c) Glucose-6-phosphat + $NADP^+$ $\xrightarrow{G6P-DH}$ Gluconat-6-phosphat + NADPH + $H^+$

tritt bisweilen eine Lag-phase auf, die bei der Messung einen Teil des Meßbereichs verbraucht und außerdem die Adaption auf bestimmte Analysenautomaten schwierig oder unmöglich macht. Diese Störung wird wie folgt beseitigt:

$$\text{Diglutathiondisulfid} + NAD(P)H + H^+ \xrightarrow{GR} 2\ \text{Glutathion} + NAD(P)^+$$

Die Durchführung der Reaktion erläutert folgende Tabelle.

Tabelle 5

| Ausgangslösung | Probe | Konz. im Test |
|---|---|---|
| Imidazolpuffer, pH 6,7, 0,2 Mol/l | 1,0 ml | 0,1 Mol/l |
| Glucose, 0,8 Mol/l | 0,05 ml | 20 mMol/l |
| Mg-acetat, 400 mMol/l | 0,05 ml | 10 mMol/l |
| ADP, 80 mMol/l | 0,05 ml | 2 mMol/l |
| AMP, 0,2 Mol/l | 0,05 ml | 5 mMol/l |
| Diadenosin-pentaphosphat, 0,4 mMol/l | 0,05 Mol | 0,01 mMol/l |
| NADP, 80 mMol/l | 0,05 ml | 2 mMol/l |
| HK, 100 U/ml | 0,05 ml | 2,5 U/ml |
| G6P-DH, 60 U/ml | 0,05 ml | 1,5 U/ml |
| GR, 20 U/ml | 0,1 ml | 1 U/ml |
| N-Acetylcystein, 400 mMol/l | 0,1 ml | 20 mMol/l |
| GSSG, 40 mMol/l | 0,1 ml | 2 mMol/l |
| Probe | 0,1 ml | |

5 min inkubieren. Während dieser Zeit läuft die Lag-phase der CK ab. Das dabei gebildete NADPH wird sofort durch GSSG/GR wieder oxidiert, so daß keine Anzeige erfolgt. Dann erfolgt der Start mit

| | | |
|---|---|---|
| Dithiothreitol, 50 mMol/l | 0,2 ml | 5 mMol/l |

Durch vollständigen Verbrauch des GSSG mit Dithiothreitol wird das Meßsignal der CK-Reaktion sichtbar, da NADPH nicht länger oxidiert wird.

Die Meßbedingungen sind für die CK-Messung optimiert. Für den erfindungsgemäßen Zusatz an GSSG, GR und Dithiothreitol gilt das unter Beispiel 1 gesagte. Die Schwankungsbreite für GSSG beträgt 0,001 mMol/l bis 200 mMol/l, bevorzugt sind 0,5 bis 5 mMol/l. GR kann von 0,02 bis 50 U/ml schwanken, 0,5 bis 5 U/ml sind bevorzugt. Das Dithiothreitol reduziert GSSG momentan zu GSH. Es muß also mindestens äquimolar zu GSSG sein. Daher beträgt die Schwankungsbreite 0,01 bis 250 mMol/l, bevorzugt sind 0,5 bis 10 mMol/l.

## Beispiel 4

### Beseitigung von endogener Glucose und Lag-phase im α-Amylase-test

Bei der Bestimmung von α-Amylase im Serum nach dem Schema

D a) $\text{Maltoheptaose} + H_2O \xrightarrow{\alpha\text{-Amylase}} \text{Triose und Tetraose}$

b) $\text{Triose} + \text{Tetraose} + 5\ H_2O \xrightarrow{\alpha\text{-Glucosidase}} 7\ \text{Glucose}$

c) $7\ \text{Glucose} + 7\ \text{ATP} \xrightarrow{\text{HK}} 7\ \text{Glucose-6-phosphat} + 7\ \text{ADP}$

d) $7\ \text{Glucose-6-phosphat} + 7\ NAD^+ \xrightarrow{\text{G6P-DH}} 7\ \text{Gluconat-6-phosphat} + 7\ \text{NADH} + 7\ H^+$

wird durch die Glucose des Serums ein großer Teil des Meßbereichs verbraucht. Die Lag-phase der α-Amylase in dieser Bestimmung verbraucht einen weiteren Teil des Meßbereichs und verhindert die Adaption an Analysenautomaten. Die Störung wird wie folgt beseitigt:

$$H_2O_2 + \text{NADH} + H^+ \xrightarrow{\text{NADH-POD}} 2\ H_2O + NAD^+$$

Die Durchführung der Reaktion erläutert folgende Tabelle.

Tabelle 6

| Ausgangslösung | Probe | Leerwert | Konz. im Test |
|---|---|---|---|
| Phosphatpuffer, pH 7, 0,1 Mol/l enthalten 0,1 Mol/l NaCl und 10 mMol/l Mg-acetat | 1,0 ml | 1,0 ml | NaCl 50 mMol/l Acetat 5 mMol/l Phosphat 50 mMol/l |
| Maltoheptaose, 0,2 Mol/l | 0,1 ml | 0,1 ml | 10 Mol/l |
| NAD, 40 mMol/l | 0,1 ml | 0,1 ml | 2 mMol/l |
| ATP, 24 mMol/l | 0,1 ml | 0,1 ml | 1,2 mMol/l |
| $H_2O_2$, 80 mMol/l | 0,05 ml | 0,05 ml | 2 mMol/l |
| HK, 80 U/ml | 0,05 ml | 0,05 ml | 2 U/ml |
| G6P-DH, 80 U/ml | 0,05 ml | 0,05 ml | 2 U/ml |
| α-Glucosidase, 1000 U/ml | 0,02 ml | 0,02 ml | 10 U/mi |
| NADH-POD, 100 U/ml | 0,01 ml | 0,01 ml | 0,5 U/ml |
| Serum | 0,02 ml | — | |
| $H_2O$ | 0,45 ml | 0,45 ml | |

Die Mischung wird 15 min inkubiert. Während der Zeit laufen endogene Glucose und Lag-phase der Amylasebestimmung ab. Das gebildete NADH wird durch $H_2O_2$/NADH-POD sofort wieder beseitigt. Dann erfolgt Start mit

| | | | |
|---|---|---|---|
| $NAHSO_3$, 0,16 Mol/l oder | 0,05 ml | 0,05 ml | 4 mMol/l oder |
| Katalase, 1 000 000 U/ml | 0,05 ml | 0,05 ml | 25 000 U/ml |

Durch $HSO_3^-$ bzw. Katalase wird momentan das $H_2O_2$ zerstört. Dadurch kann das gebildete NADH nicht mehr abgebaut werden und somit die α-Amylase-aktivität sofort und ohne Störung gemessen werden. Anstelle von $HSO_3^-$ oder Katalase kann auch Glutathion/Glutathion-peroxidase zugesetzt werden. Die Reaktionsbedingungen werden von den optimalen Bedingungen der α-Amylase bestimmt.

Für den erfindungsgemäßen Zusatz von $H_2O_2$, NADH-POD und $NaHSO_3$ bzw. Katalase gilt das unter Beispiel 1 gesagte.

Die $H_2O_2$-Konzentration kann von 0,01 bis 50 mMol/l reichen, bevorzugt sind 1 bis 5 mMol/l. Die NADH-POD-Konzentration kann 0,001 bis 100 U/ml betragen, bevorzugt sind 0,1 bis 5 U/ml. $HSO_3$ muß mindestens äquimolar zu $H_2O_2$ zugesetzt werden, daher sind 0,001 bis 250 mMol/l möglich. Bevorzugt werden 1 bis 10 mMol/l. Für Katalase liegt der Bereich, der gewählt werden kann, zwischen 10 bis 100 000 U/ml, bevorzugt sind 500 bis 50 000 U/ml.

Abbildung 1 der beigefügten Zeichnung zeigt einen typischen Kurvenverlauf, den man mit und ohne Anwendung der Erfindung erhält. Kurve 1, ohne den erfindungsgemäßen Zusatz von NADH-POD/$H_2O_2$ und Katalase, läßt den durch endogene Glucose bewirkten starken Verlauf erkennen, der dann in die Lag-phase der α-Amylase übergeht. Erst nach 14 min ( ↓ ) ist die Linearität erreicht. Kurve 2 zeigt im ersten Teil den Abbau des aus endogener Glucose gebildeten NADH; nach Erreichen der Grundextinktion ändert sich der Wert bis zum Start ( ↑ ) mit Katalase nicht mehr. Dann allerdings beginnt sofort der lineare Verlauf der α-Amylase-reaktion, der direkt gemessen werden kann.

## Beispiel 5

### Beseitigung von endogener Glucose und Lag-phase im α-Amylase-test

Bei der Beseitigung der α-Amylase-aktivität nach folgendem Schema

E a) $\text{Maltoheptaose} + H_2O \xrightarrow{\alpha\text{-Amylase}} \text{Triose und Tetraose}$

b) $\text{Triose} + \text{Tetraose} + 5\ H_2O \xrightarrow{\alpha\text{-Glucosidase}} 7\ \text{Glucose}$

c) $7\ \text{Glucose} + 7\ NAD^+ \xrightarrow{\text{Gluc-DH}} 7\ \text{Gluconat} + 7\ NADH + 7\ H^+$

treten dieselben Störmöglichkeiten, wie im Beispiel 4 beschrieben, auf. Sie werden wie folgt beseitigt:

$$\text{Fructose} + \text{NADH} + H^+ \xrightarrow{\text{SDH}} \text{Sorbit} + \text{NAD}^+$$

Die Durchführung der Reaktion erläutert folgende Tabelle.

Tabelle 7

| Ausgangslösung | Probe | Leerwert | Konz. im Test |
|---|---|---|---|
| Phosphatpuffer, pH 7,0 | 1,0 ml | 1,0 ml | Phosphat 50 mMol/l |
| 0,1 Mol/l, enthaltend | 1,0 ml | 1,0 ml | Maltoheptaose |
| Maltoheptaose 20 mMol/l | 1,0 ml | 1,0 ml | |
| NaCl 0,1 Mol/l | 1,0 ml | 1,0 ml | NaCl 50 mMol/l |
| $MgCl_2$ 4 mMol/l | 1,0 ml | 1,0 ml | $MgCl_2$ 2 mMol/l |
| NAD 40 mMol/l | 0,1 ml | 0,1 ml | 2 mMol/l |
| Glucose-dehydrogenase 360 u/ml | 0,1 ml | 0,1 ml | 18 U/ml |
| Fructose 0,6 Mol/l | 0,1 ml | 0,1 ml | 30 mMol/l |
| SDH 3 U/ml | 0,1 ml | 0,1 ml | 0,15 U/ml |
| Serum | 0,02 ml | — | |
| $H_2O$ | 0,5 ml | 0,5 ml | |

Die Mischung wird 15 min inkubiert. Während der Zeit laufen endogene Glucose und Lag-phase der $\alpha$-Amylasebestimmung ab. Das gebildete NADH wird durch Fructose-SDH sofort wieder beseitigt. Dann erfolgt Start mit

| | | | |
|---|---|---|---|
| 2,3-Dimercapto-1-propanol 40 mMol/l | 0,1 ml | 0,1 ml | 2 mMol/l |

Durch Zugabe von 2,3-Dimercapto-1-propanol wird die SDH momentan quantitativ gehemmt. Dadurch kann das gebildete NADH nicht mehr abgebaut werden und somit die $\alpha$-Amylase-aktivität sofort und ohne Störung gemessen werden. Abbildung 2 der beigefügten Zeichnung zeigt einen typischen Kurvenverlauf, den man mit und ohne Anwendung der Erfindung erhält. Kurve I ohne den erfindungsgemäßen Zusatz von Fructose/SDH und 2,3-Dimercapto-1-propanol zeigt deutlich den durch die endogene Glucose verursachten, starken Verlauf, der zu Extinktionen über 1 führt und nach ca. 15 min (↓) abgeschlossen ist und in den linearen Bereich der $\alpha$-Amylase-reaktion übergeht. Kurve 2 mit den erfindungsgemäßen Zusätzen von Fructose/SDH zeigt im ersten Teil noch den Abbau des aus endogener Glucose gebildeten NADH. Nach Erreichen des Grundwerts ändert sich die Extinktion bis zum Start mit 2,3-Mercapto-1-propanol 1(˙) nicht mehr. Danach kann sofort aus dem linearen Kurvenverlauf die $\alpha$-Amylase-aktivität abgelesen und berechnet werden.

Die Reaktionsbedingungen werden von den optimalen Bedingungen der $\alpha$-Amylase-reaktion bestimmt. Für den erfindungsgemäßen Zusatz von Fructose-SDH und 2,3-Dimercapto-1-propanol gelten die Angaben in Beispiel 1. Die zulässigen Konzentrationen entsprechen denen von Beispiel 2.

**Patentansprüche**

1. Verfahren zur Durchführung enzymatischer Bestimmungen, die unter Bildung von NAD(P)H verlaufen und bei denen Zwischenprodukte und unspezifisch reagierende Störsubstanzen in einer Vorreaktion abreagieren gelassen werden, dadurch gekennzeichnet, daß man während der Vorreaktion gleichzeitig eine NAD(P)H-verbrauchende enzymatische Reaktion mitführt, die das gebildete NAD(P)H vollständig zu NAD(P) oxidiert und zum Start der Meßreaktion die NAD(P)H-verbrauchende Reaktion gestoppt wird.

2. Verfahren nach Anspruch 1, dadurch gekennzeichnet, daß die NAD(P)H-verbrauchende Reaktion durch Zusatz eines Inhibitors für diese Reaktion gestoppt wird.

3. Verfahren nach Anspruch 1, dadurch gekennzeichnet, daß die NAD(P)H-verbrauchende Reaktion durch Abfangen des zweiten Substrats dieser Reaktion gestoppt wird.

4. Verfahren nach Anspruch 2, dadurch gekennzeichnet, daß man als NAD(P)H-verbrauchende Reaktion die Umsetzung mit Pyruvat in Gegenwart von Lactatdehydrogenase (LDH) anwendet und

durch Zusatz des LDH-Inhibitors aus NADH die Reaktion stoppt.

5. Verfahren nach Anspruch 2, dadurch gekennzeichnet, daß man als NAD(P)H-verbrauchende Reaktion die Umsetzung mit Fructose in Gegenwart von Sorbitdehydrogenase anwendet und die Reaktion durch Zusatz von 2,3-Dithiol-1-propanol stoppt.

6. Verfahren nach Anspruch 3, dadurch gekennzeichnet, daß man als NAD(P)H-verbrauchende Reaktion die Umsetzung mit Glutathiondisulfid in Gegenwart von Glutathion-reductase anwendet und die Reaktion durch Zusatz von Dithiothreitol abstoppt.

7. Verfahren nach Anspruch 3, dadurch gekennzeichnet, daß man als NADH-verbrauchende Reaktion die Umsetzung mit Wasserstoffperoxid in Gegenwart von NADH-peroxidase anwendet und die Reaktion durch Zusatz von Katalase oder Sulfit oder Glutathion/Glutäthinon-peroxidase abstoppt.

8. Reagens zur Durchführung enzymatischer Bestimmungen, enthaltend ein System zur Bestimmung eines Substrats oder Enzyms, welches zur Bildung von NAD(P)H führt, dadurch gekennzeichnet, daß es zusätzlich ein enzymatisches System enthält, welches NAD(P)H vollständig zu NAD(P) oxidiert.

9. Reagens nach Anspruch 8, dadurch gekennzeichnet, daß das System, welches NAD(P)H oxidiert, aus einer Dehydrogenase oder Reductase und ihrem von NAD(P)H verschiedenen Substrat besteht.

10. Reagens nach Anspruch 9, dadurch gekennzeichnet, daß das System aus Lactatdehydrogenase und Pyruvat besteht.

11. Reagens nach Anspruch 9, dadurch gekennzeichnet, daß das System aus Sorbitdehydrogenase und Fructose besteht.

12. Reagens nach Anspruch 9, dadurch gekennzeichnet, daß das System aus Glutathionreductase und Diglutathiondisulfid besteht.

13. Reagens nach Anspruch 9, dadurch gekennzeichnet, daß das System aus NADH-Peroxidase und $H_2O_2$ oder einer $H_2O_2$ liefernden Verbindung besteht.

14. Testkit zur Durchführung des Verfahrens nach einem der Ansprüche 1 bis 7, enthaltend ein Reagens nach einem der Ansprüche 8 bis 13 und einem Inhibitor für das NAD(P)H oxidierende System.

15. Testkit nach Anspruch 14, enthaltend ein Reagens nach Anspruch 10 und LDH-Inhibitor.

16. Testkit nach Anspruch 14, enthaltend ein Reagens nach Anspruch 11 und 2,3-Dithiol-1-propanol.

17. Testkit nach Anspruch 14, enthaltend ein Reagens nach Anspruch 12 und Dithiotreitol.

18. Testkit nach Anspruch 14, enthaltend ein Reagens nach Anspruch 13 und Sulfid oder Glutathion und Glutathionperoxidase.

19. Testkit nach Anspruch 15 zur Bestimmung von Triglycerid nach dem Verfahren von Anspruch 4, bestehend aus NAD, Glycerin-dehydrogenase, Aldehyddehydrogenase, Pyruvat, LDH und Puffer und getrennt davon Esterase und LDH-Inhibitor.

20. Testkit nach Anspruch 16 zur Bestimmung von Creatinin im Serum nach dem Verfahren von Anspruch 5, enthaltend NADP, ATP, Phosphoenolpyruvat, Glutamat, $\alpha$-Aminobutyrat, Magnesiumchlorid, Fructose, Creatinkinase, Pyruvatkinase, Sorbitdehydrogenase, Glutamat-pyruvat-transaminase, $\gamma$-Aminobutyrat-transaminase, Succinat-semialdehyd-dehydrogenase und getrennt davon Creatininase und 2,3-Dithiol-1-propanol.

21. Testkit nach Anspruch 17 zur Bestimmung der Creatinkinase nach dem Verfahren von Anspruch 6, dadurch gekennzeichnet, daß er Puffer, Glucose, Magnesiumacetat, ADP, AMP, Diadenosinpentaphosphat, NADP, Hexokinase, Glucose-6-phosphat-dehydrogenase, Glutathionreductase, N-Acetylcystein und Diglutathiondisulfid und getrennt davon Dithiotreitol enthält.

22. Testkit nach Anspruch 18 zur $\alpha$-Amylasebestimmung nach dem Verfahren von Anspruch 7, enthaltend Puffer, NaCl, Magnesiumacetat, Maltoheptaose, NAD, ATP, $H_2O_2$, Hexokinase, Glucose-6-phosphat-dehydrogenase, $\alpha$-Glucosidase, NADH-Peroxidase und getrennt davon $NaHSO_3$ oder Katalase.

23. Testkit nach Anspruch 16 zur Bestimmung von $\alpha$-Amylase nach dem Verfahren von Anspruch 5, enthaltend Puffer, NaCl, $MgCl_2$, Maltoheptaose, NAD, Glucosedehydrogenase, Fructose, Sorbitdehydrogenase und getrennt davon 2,3-Dimercapto-1-propanol.

**Claims**

1. Process for the carrying out of enzymatic determinations which proceed with the formation of NAD(P)H and in which intermediate products and nonspecifically reacting disturbing substances are allowed to react to completion in a preliminary reaction, characterised in that during the preliminary reaction one simultaneously carries out an enzymatic reaction consuming NAD(P)H which completely oxidises the NAD(P)H formed to NAD(P) and, at the commencement of the measurement reaction, the reaction consuming NAD(P)H is stopped.

2. Process according to claim 1, characterised in that the reaction consuming NAD(P)H is stopped by the addition of an inhibitor for this reaction.

3. Process according to claim 1, characterised in that the reaction consuming NAD(P)H is stopped

by removal of the second substrate of this reaction.

4. Process according to claim 2, characterised in that as reaction consuming NAD(P)H one employs the reaction with pyruvate in the presence of lactate dehydrogenase (LDH) and stops the reaction by the addition of the LDH inhibitor from NADH.

5. Process according to claim 2, characterised in that as reaction consuming NAD(P)H one employs the reaction with fructose in the presence of sorbitol dehydrogenase and stops the reaction by the addition of 2,3-dithiolpropan-1-ol.

6. Process according to claim 3, characterised in that as reaction consuming NAD(P)H one employs the reaction with glutathione disulphide in the presence of glutathione reductase and stops the reaction by the addition of dithiothreitol.

7. Process according to claim 3, characterised in that as reaction consuming NADH one employs the reaction with hydrogen peroxide in the presence of NADH peroxidase and stops the reaction by the addition of catalase or sulphite or glutathione/glutathione peroxidase.

8. Reagent for the carrying out of enzymatic determinations, containing a system for the determination of a substrate or enzyme which leads to the formation of NAD(P)H, characterised in that it additionally contains an enzymatic system which completely oxidises NAD(P)H to NAD(P).

9. Reagent according to claim 8, characterised in that the system which oxidises NAD(P)H consists of a dehydrogenase or reductase and its substrate which is different from NAD(P)H.

10. Reagent according to claim 9, characterised in that the system consists of lactate dehydrogenase and pyruvate.

11. Reagent according to claim 9, characterised in that the system consists of sorbitol dehydrogenase and fructose.

12. Reagent according to claim 9, characterised in that the system consists of glutathione reductase and diglutathione disulphide.

13. Reagent according to claim 9, characterised in that the system consists of NADH peroxidase and $H_2O_2$ or of an $H_2O_2$-yielding compound.

14. Test kit for the carrying out of the process according to one of claims 1 to 7, containing a reagent according to one of claims 8 to 13 and an inhibitor for the NAD(P)H-oxidising system.

15. Test kit according to claim 14, containing a reagent according to claim 10 and LDH inhibitor.

16. Rest kit according to claim 14, containing a reagent according to claim 11 and 2,3-dithiopropan-1-ol.

17. Test kit according to claim 14, containing a reagent according to claim 12 and dithiothreitol.

18. Test kit according to claim 14, containing a reagent according to claim 13 and sulphite or glutathione or glutathione peroxidase.

19. Test kit according to claim 15 for the determination of triglyceride according to the process of claim 4, consisting of NAD, glycerol dehydrogenase, aldehyde dehydrogenase, pyruvate, LDH and buffer and, separately therefrom, esterase and LDH inhibitor.

20. Test kit according to claim 16 for the determination of creatinine in serum according to the process of claim 5, containing NADP, ATP, phosphoenol pyruvate, glutamate, $\alpha$-aminobutyrate, magnesium chloride, fructose, creatine kinase, pyruvate kinase, sorbitol dehydrogenase, glutamate-pyruvate transaminase, $\gamma$-aminobutyrate transaminase, succinate semialdehyde dehydrogenase and, separately therefrom, creatininase and 2,3-dithiolpropan-1-ol.

21. Test kit according to claim 17 for the determination of creatine kinase according to the process of claim 6, characterised in that it contains buffer, glucose, magnesium acetate, ADP, AMP, diadenosine pentaphosphate, NADP, hexokinase, glucose-6-phosphate dehydrogenase, glutathione reductase, N-acetylcysteine and diglutathione disulphide and, separately therefrom, dithiothreitol.

22. Test kit according to claim 18 for the determination of $\alpha$-amylase according to the process of claim 7, containing buffer, NaCl, magnesium acetate, maltoheptaose, NAD, ATP, $H_2O_2$, hexokinase, glucose-6-phosphate dehydrogenase, $\alpha$-glucosidase, NADH peroxidase and, separately therefrom, $NaHSO_3$ or catalase.

23. Test kit according to claim 16 for the determination of $\alpha$-amylase according to the process of claim 5, containing buffer, NaCl, $MgCl_2$, maltoheptaose, NAD, glucose dehydrogenase, fructose, sorbitol dehydrogenase and, separately therefrom, 2,3-dimercaptopropan-1-ol.

**Revendications**

1. Procédé pour effectuer des dosages enzymatiques qui se déroulent avec formation de NAD(P)H et selon lesquels on laisse réagir complètement, dans une réaction préalable, des produits intermédiaires et des substances perturbatrices réagissant de façon non spécifique, caractérisé en ce que, pendant la réaction préalable on effectue simultanément une réaction enzymatique consommant du NAD(P)H, laquelle réaction oxyde complètement le NAD(P)H en NAD(P) et on arrête la réaction consommant du NAD(P)H au début de la réaction de mesure.

2. Procédé selon la revendication 1, caractérisé en ce que la réaction consommant du NAD(P)H est arrêtée par addition d'un inhibiteur de cette réaction.

3. Procédé selon la revendication 1, caractérisé en ce que la réaction consommant du NAD(P)H est arrêtée par capture du deuxième substrat de cette réaction.

4. Procédé selon la revendication 2, caractérisé en ce qu'on utilise, en tant que réaction consommant du NAD(P)H, la réaction avec le pyruvate en présence de lactatedeshydrogénase (LDH) et on arrête la réaction par addition de l'inhibiteur de LDH à partir de NADH.

5. Procédé selon la revendication 2, caractérisé en ce qu'on utilise, en tant que réaction consommant du NAD(P)H, la réaction avec le fructose en présence de sorbitoldeshydrogénase et on arrête la réaction par addition de 2,3-dithiol-1-propanol.

6. Procédé selon la revendication 3, caractérisé en ce qu'on utilise, en tant que réaction consommant du NAD(P)H, la réaction avec le bisulfure de glutathion en présence de gluathion—réductase et on arrête la réaction par addition de dithiothréitol.

7. Procédé selon la revendication 3, caractérisé en ce qu'on utilise, en tant que réaction consommant du NADH, la réaction avec le peroxyde d'hydrogène en présence de NADH-peroxydase et on arrête la réaction par addition de catalase ou de sulfite ou de glutathion/glutéthinone-peroxydase.

8. Réactif pour effectuer des dosages enzymatiques, comprenant un système pour le dosage d'un substrat ou enzyme, lequel conduit à la formation de NAD(P)H, caractérisé en ce qu'il comprend en outre un système enzymatique, lequel oxyde complètement le NAD(P)H en NAD(P).

9. Réactif selon la revendication 8, caractérisé en ce que le système qui oxyde le NAD(P)H est constitué d'une deshydrogénase ou réductase et de son substrat différent du NAD(P)H.

10. Réactif selon la revendication 9, caractérisé en ce que le système est constitué de lactate des hydrogénase et de pyruvate.

11. Réactif selon la revendication 9, caractérisé en ce que le système est constitué de sorbitol des hydrogénase et de fructose.

12. Réactif selon la revendication 9, caractérisé en ce que le système est constitué de glutathion réductase et de bisulfure de diglutathion.

13. Réactif selon la revendication 9, caractérisé en ce que le système est constitué de NADH-peroxydase et de $H_2O_2$ ou d'un composé fournissant $H_2O_2$.

14. Nécessaire pour essai (»kit pour test«) pour effectuer le procédé selon l'une des revendications 1 à 7, comprenant un réactif selon l'une des revendications 8 à 13 et un inhibiteur pour le système oxydant le NAD(P)H.

15. Nécessaire pour essai (»kit pour test«) selon la revendication 14, comprenant un réactif selon la revendication 10 et de l'inhibiteur de LDH.

16. Nécessaire pour essai (»kit pour test«) selon la revendication 14, comprenant un réactif selon la revendication 11 et du 2,3-dithiol-1-propanol.

17. Nécessaire pour essai (»kit pour test«) selon la revendication 14, comprenant un réactif selon la revendication 12 et du dithiotréitol.

18. Nécessaire pour essai (»kit pour test«) selon la revendication 14, comprenant un réactif selon la revendication 13 et du sulfure ou du glutathion et de la glutathionperoxydase.

19. Nécessaire pour essai (»kit pour test«) selon la revendication 15 pour le dosage d'un triglycéride selon le procédé de la revendication 4, constitué de NAD, de glycérine-deshydrogénase, d'aldéhyde-deshydrogénase, de pyruvate, de LDH et de tampon et, séparément, d'estérase et d'inhibiteur de LDH.

20. Nécessaire pour essai (»kit pour test«) selon la revendication 16 pour le dosage de la créatinine dans le sérum selon le procédé de la revendication 5, comprenant du NADP, de l'ATP, du phosphoénolpyruvate, du glutamate, de l'$\alpha$-aminobutyrate, du chlorure de magnésium, du fructose, de la créatinekinase, de la pyruvatekinase, de la sorbitoldeshydrogénase, de la glutamate-pyruvate-transaminase, de la $\gamma$-aminobutyrate-transaminase, de la succinate-semialdéhydedeshydrogénase et, séparément, de la créatininase et du 2,3-dithiol-1-propanol.

21. Nécessaire pour essai (»kit pour test«) selon la revendication 17 pour le dosage de la créatinekinase selon le procédé de la revendication 6, caractérisé en ce qu'il comprend du tampon, du glucose, de l'acétate de magnésium, de l'ADP, de l'AMP, du pentaphosphate de diadénosine, du NADP, de l'hexokinase, de la glucose-6-phosphate-deshydrogénase, de la glutathionréductase, de la N-acétylcystéine et du bisulfure de diglutathion et, séparément, du dithiotréitol.

22. Nécessaire pour essai (»kit pour test«) selon la revendication 18 pour le dosage de l'$\alpha$-amylase selon le procédé de la revendication 7, comprenant du tampon, du NaCl, de l'acétate de magnésium, du maltoheptaose, du NAD, de l'ATP, du $H_2O_2$, de l'hexokinase, de la glucose-6-phosphatedes-hydrogénase, de l'$\alpha$-glucosidase, de la NADH-peroxydase et, séparément, du $NaHSO_3$ ou de la catalase.

23. Nécessaire pour essai (»kit pour test«) selon la revendication 16 pour le dosage de l'$\alpha$-amylase selon le procédé de la revendication 5, comprenant du tampon, du NaCl, du $MgCl_2$, du maltoheptaose, du NAD, de la glucosedeshydrogénase, du fructose, de la sorbitoldeshydrogénase et, séparément, du 2,3-dimercapto-1-propanol.

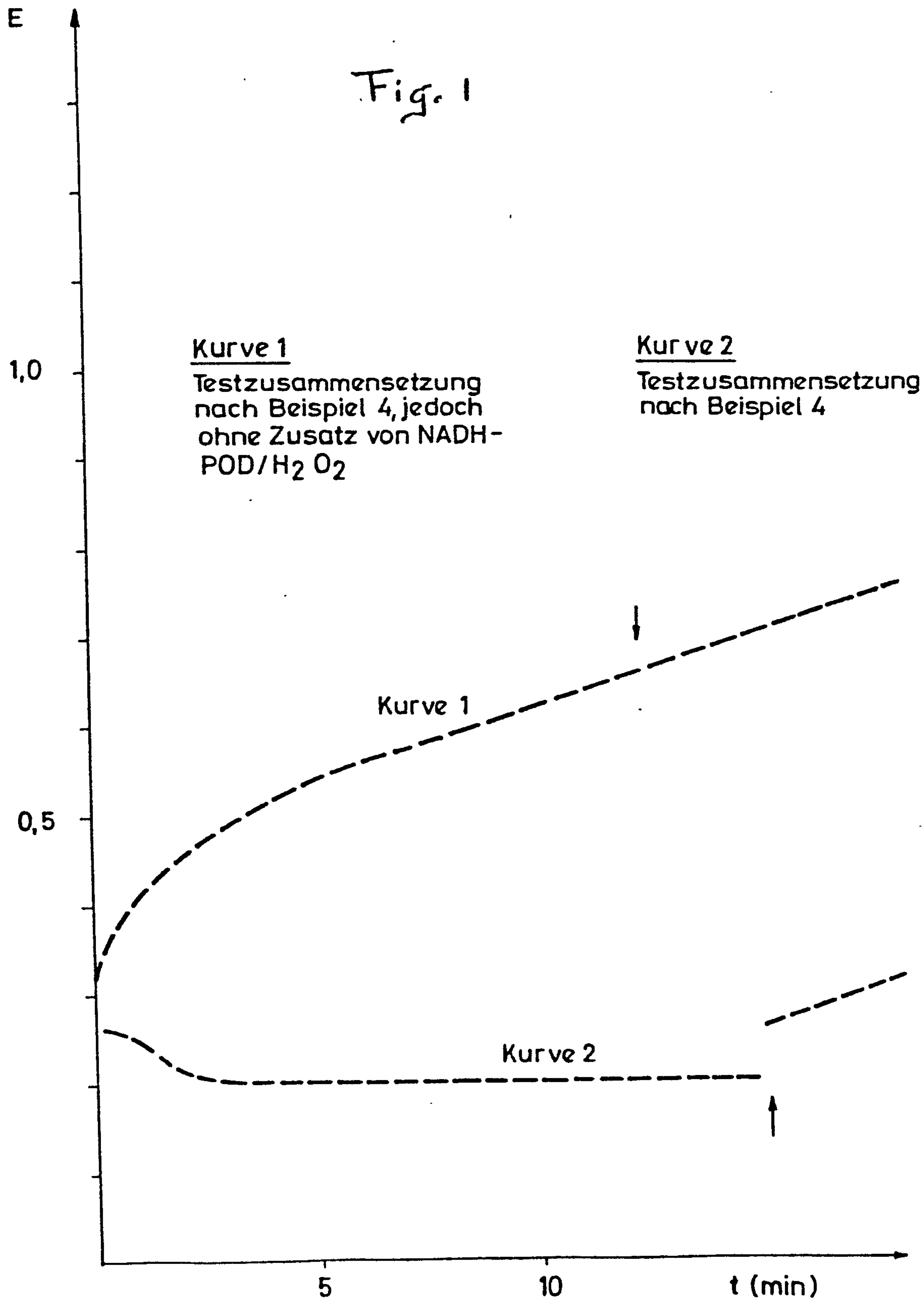
E
Fig. 1
1,0
0,5
Kurve 1
Testzusammensetzung nach Beispiel 4, jedoch ohne Zusatz von NADH-POD/$H_2O_2$
Kurve 2
Testzusammensetzung nach Beispiel 4
Kurve 1
Kurve 2
5
10
t (min)

Fig. 2

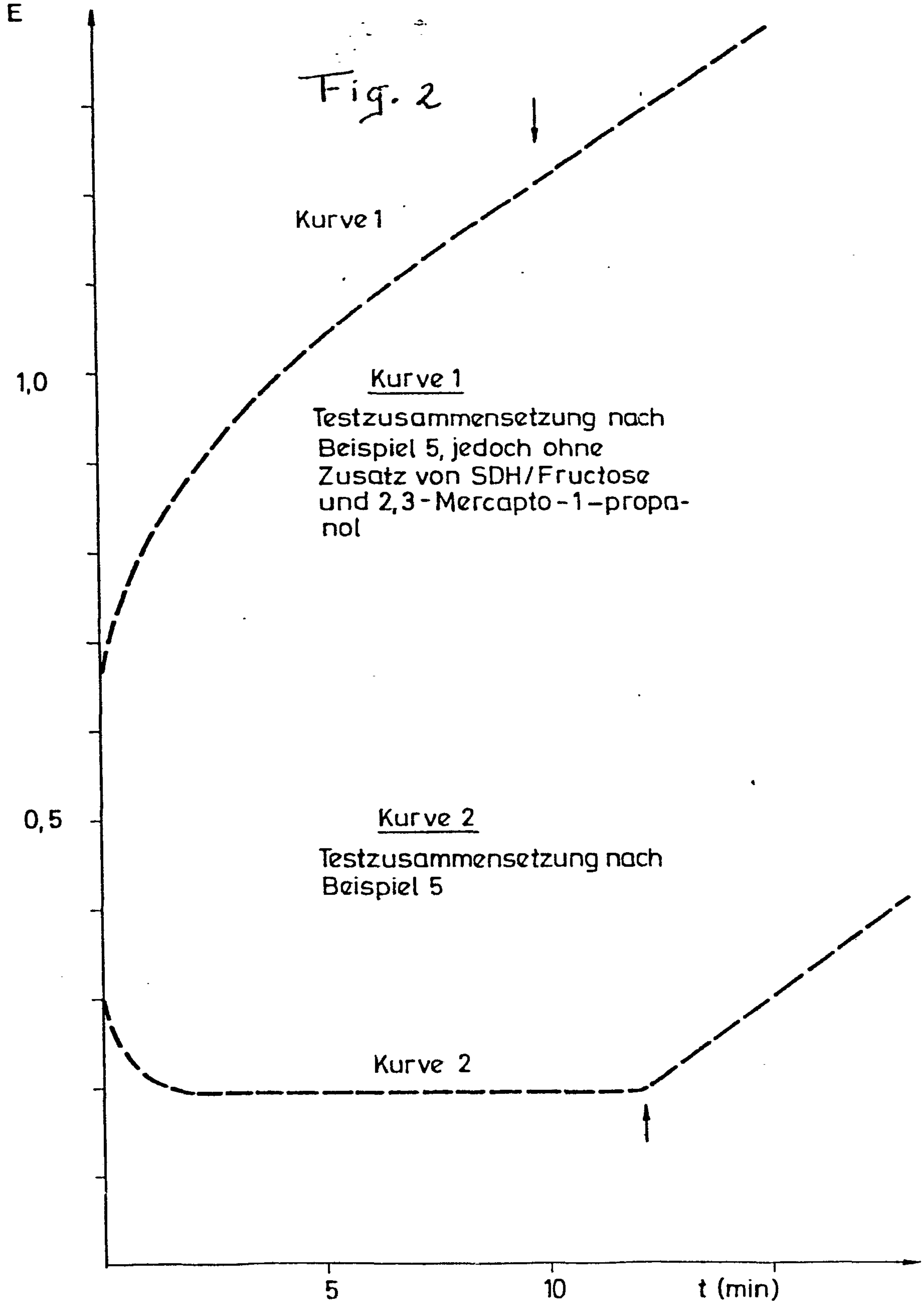
E
Kurve 1
1,0
Kurve 1
Testzusammensetzung nach
Beispiel 5, jedoch ohne
Zusatz von SDH/Fructose
und 2,3-Mercapto-1-propa-
nol
0,5
Kurve 2
Testzusammensetzung nach
Beispiel 5
Kurve 2
5
10
t (min)